(19) Europäisches Patentamt
European Patent Office
Office européen des brevets

(11) **EP 4 354 137 A1**

(12) **EUROPEAN PATENT APPLICATION**

(43) Date of publication:
**17.04.2024 Bulletin 2024/16**

(21) Application number: **22382988.8**

(22) Date of filing: **14.10.2022**

(51) International Patent Classification (IPC):
***G01N 33/18*** *(2006.01)*

(52) Cooperative Patent Classification (CPC):
**G01N 33/1853; A47J 31/00; A47L 15/4297; D06F 34/22;** A21B 3/04; A47L 2401/30; D06F 2103/44; F24C 15/327

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
• **BSH Hausgeräte GmbH**
**81739 München (DE)**
• **BSH Electrodomésticos España, S.A.**
**50016 Zaragoza (ES)**

(72) Inventors:
• **Blecua, Aurora**
**50008 Zaragoza (ES)**

• **Calabuig Adobes, Irene**
**Zaragoza (ES)**
• **Garcia Muro, Irene**
**50007 Zaragoza (ES)**
• **Perez Nicolas, Maria**
**50007 Zaragoza (ES)**
• **Rojo Esteban, Lander**
**50009 Zaragoza (ES)**
• **Tur Gil, Alejandro Juan**
**28029 Madrid (ES)**

Remarks:
Amended claims in accordance with Rule 137(2) EPC.

(54) **A METHOD OF DETERMINING A WATER HARDNESS LEVEL OF WATER AND A HOUSEHOLD APPLIANCE HAVING AT LEAST ONE COMPONENT SUPPLIED WITH WATER DURING OPERATION**

(57) The invention relates to a method of determining a water hardness level of water, especially for determining the water hardness of water used by a household appliance (1), wherein
- at least two electrodes (16) are exposed to the water;
- an electric voltage acts on the at least two electrodes (16), wherein the electric voltage is an alternating electric voltage having a predetermined frequency range, wherein the frequency range is provided by a frequency sweep;
- in response to the electric voltage acting on the electrodes, respective electric currents are measured for the different frequencies of the frequency sweep, and
- respective electric impedance values are calculated for the frequencies based on the electric voltage and the electric currents measured, wherein the electric impedance values are processed by an analyzing apparatus (13) connected with the electrodes (16) which is configured to use a machine-learning model based on supervised learning.

Fig.21

**Description**

The invention relates to a method of determining a water hardness level of water, especially for determining the water hardness of water used by a household appliance, wherein at least two electrodes are exposed to the water, an electric voltage acts on the at least two electrodes, wherein the electric voltage is an alternating electric voltage having a predetermined frequency range, wherein the frequency range is provided by a frequency sweep comprising plural different frequencies of the frequency range, in response to the electric voltage acting on the electrodes, respective electric currents are measured for the different frequencies of the frequency sweep, and respective electric impedance values are calculated for the plural frequencies of the frequency sweep based on the electric voltage and the electric currents measured. Moreover, the invention relates to a method of training an analyzing apparatus configured to determine a water hardness of water. Furthermore, the invention also relates to a household appliance, especially a washing machine, a dishwasher, a coffee machine, or a steam oven, having at least one component, which is supplied with water during operation of the household appliance, at least two electrodes which are arranged to contact the water, a current sensor for sensing an electric current passing the electrodes and a control apparatus configured to control the operation of the household appliance, wherein the control apparatus is electrically connected with the at least two electrodes and the control apparatus is configured to determine a water hardness level of the water. Finally, the invention relates to a computer program product and a computer readable medium.

There are various household appliances which use water during the operation. For example, in a household appliance configured as a steam oven, water is heated to produce steam or vapor which is then introduced into an oven chamber of the steam oven. In another household appliance configured for example as a coffee machine, water is heated in order to prepare coffee, and in still another household appliance, such as a washing machine or a dishwasher, water is heated for washing purposes. Heating of water in such household appliances or household devices usually leads to the formation of lime or lime scale on surfaces of the components which are in contact with the water during operation of the household appliance.

However, there are also components such as a water tank, a pipe, or a pump, which are substantially in continuous contact with the water during operation of the household appliance, even if in these household appliances, the water is not heated. Even in such household appliances, in which water is not actively heated during operation, the formation of lime scale can occur due to a vaporization of water, or the like.

Lime scale deposits on or in the respective components of household appliances are unfavorable because of associated problems such as an at least partial obstruction of pipes or the like, a decrease in heat transfer or a lack of hygiene.

Generally, the household appliances may have an input device allowing a user manually entering a value of the water hardness of the water. However, the water hardness level usually depends on the hardness of the tap water which is available at a specific location where the household appliance is operated or located. The water hardness level manually entered by the user can be taken into account by a control apparatus of the household appliance. Based on the manually entered water hardness level, the household appliance can then recommend to perform a lime removal action from time to time. Such recommendation can depend on the water hardness level entered manually by the user and on incidence of use of the household appliance. However, relying on the water hardness level entered by the user, entering the water hardness level by the user is cumbersome and also prone to error. Moreover, by the time, the water hardness level of the water may vary. This also may effect lime scale deposition.

Some household appliances teach using determining an electric conductivity of water in order to determine the water hardness level. In this regard, JP 5-707246 A teaches a washing machine having a function of measuring a water hardness used in particular for washing. However, the method described in this disclosure is inaccurate and only allows an insufficient performance.

However, water hardness is also a problem with regard to cleaning performance efficiency. In locations, where the water hardness is high, the cleaning performance is reduced, for instance, due to an interaction between $Ca^{++}$-ions and some ingredients of the household appliance during its operation. Moreover, lime deposition may cause a problem with regard to energy consumption. Lime deposition on a heating element may increase energy consumption due to a decrease of a thermal conductivity to the water to be heated. For instance, lime scale may affect the heat transfer in heating elements. Moreover, a problem of glass corrosion may occur. In this regard, ion exchange may be used in order to avoid lime deposition in the household appliance. The water is softened by such an ion exchange, containing, for example, $Na^{+}$-ions. In this regard, the problem of deposition of lime on components may be reduced and the cleaning performance may be improved. However, this method rises a risk of a very low water hardness level, which may be then prone to glass corrosion. Further problems may occur with regard to a flow reduction in pipe lines, more frequent maintenance, shorter life time, and/or the like.

Therefore, is an object of the invention, to improve an accuracy of determining a water hardness level, especially with regard to a household appliance.

According to the invention, methods and a household appliance according to the independent claims are proposed. Moreover, a computer program product and a computer readable medium are proposed.

Preferable embodiments can be derived from the features of the dependent claims.

According to a first method-related aspect, which is directed to a generic method of determining a water hardness level of water, it is especially proposed that the electric impedance values are processed by an analyzing apparatus connected with the electrodes which is configured to use a machine-learning model based on supervised learning, in order to determine the water hardness level.

According to a second method-related aspect of training an analyzing apparatus according to the invention, it is especially proposed that training data is generated by using the analyzing apparatus under predefined conditions, wherein the method comprises the steps of providing different water samples having water hardness levels differing from each other, for each of the different water samples, measuring an amount of lime deposition on electrodes connected with the analyzing apparatus dependent on the different water samples under the predefined conditions; subjecting the electrodes to an electric voltage, wherein the electric voltage is an alternating electric voltage having a predetermined frequency range, wherein the frequency range is provided by a frequency sweep comprising plural different frequencies of the frequency range, measuring electric currents responsive to the electric voltage for each of the plural different frequencies of the frequency sweep; and calculating electric impedances dependent on the plural different frequencies of the frequency sweep resulting in respective plural frequency dependent electric impedance values, processing the different water samples and the respective lime deposition amounts in order to identify a correlation between the different water samples and the respective lime deposition amounts; processing the plural frequency dependent electric impedance values of the frequency sweeps and the respective lime deposition amounts, which depend on the water samples, in order to identify a correlation between the plural frequency dependent electric impedance values of the frequency sweeps and the respective lime deposition amounts dependent on the water hardness levels of the different water samples; generating the training data based on the identified correlation between the plural frequency dependent electric impedance values of the frequency sweeps and the respective different water samples in order to train the analyzing apparatus; and applying the training data on a machine-learning model using supervised learning.

According to a third household appliance-related aspect, it is especially proposed that the control apparatus comprises an analyzing apparatus connected with the electrodes, the analyzing apparatus being configured to use a machine-learning model based on supervised learning, in order to determine the water hardness level, wherein the control apparatus is further configured to supply an electric voltage to the at least two electrodes, wherein the electric voltage is an alternating electric voltage having a predetermined frequency range, wherein the frequency range is provided by a frequency sweep comprising plural different frequencies of the frequency range; in response to the electric voltage acting on the electrodes, measure electric currents with the current sensor responsive to the electric voltage for each of the plural different frequencies; calculate respective electric impedance values for the plural frequencies of the frequency sweep based on the electric voltage and the electric currents measured; and process the electric impedance values by the analyzing apparatus connected with the electrodes in order to determine the water hardness level.

According to a fourth computer-related aspect, a computer program product comprising instructions is proposed, which, when the computer program is executed by a computer, cause the computer to carry out at least one of the inventive methods.

According to a fifth computer-related aspect, a computer readable medium comprising instructions is proposed which, when executed by a computer, cause the computer to carry out at least one of the inventive methods.

The invention is beside others based on the idea that determining the water hardness level of water can be improved by using impedance spectroscopy combined with machine-learning. In this regard, the impedance spectroscopy allows generating a specific raw data set for a specific water hardness level of water indicative of this water hardness level. However, it should be noted that, as will be also detailed below, the impedance values resulting from the impedance spectroscopy may also be dependent on a lime deposition in the household appliance, especially on the electrodes. Therefore, the invention considers that impedance spectroscopy itself could be not sufficient in order to identify the water hardness level of the water with a desired high accuracy. In this regard, the inventors found that the accuracy can be enhanced by using a machine-learning algorithm which is preferably trained with specific training data considering different lime scale amounts and water hardness levels. In this regard, the analysing apparatus is enabled to determine the water hardness level with a high accuracy because of its training with the training data which are also part of the invention as further detailed below.

In this regard, the invention allows determining the water hardness level with high accuracy wherein complex and expensive components can be substantially avoided which is advantageous for the use in household appliances.

The control apparatus may be integral with the analysing apparatus or it may be a separate component of the control apparatus. The analysing apparatus and/or the control apparatus are established as a hardware circuitry, a computer running a program, or the like. The analysing apparatus may be a hardware circuitry, a computer running a program, combinations thereof, or the like. So, the analysing apparatus and/or the control apparatus may also be provided as a chip such as a semiconductor chip which may form a component of the household appliance or any other device, or it may be integral therewith.

The electric voltage has a predetermined amplitude which, for instance, may have a value in a range of about 0.01 V to about 1 V, preferably in a range of about 0.08 V to about 0.15 V, most preferably about 0.1 V. The frequency sweep may comprise a plurality of different discrete frequencies of the frequency range. The frequencies may be equal distant or may be chosen with the varying distances. The number of the frequencies may also vary. The number may be, for instance, at least 5. Preferably, the number of the frequencies may be more than 100. Most preferably, the frequencies of the sweep may provide a nearly continuous spectrum of the frequencies of the frequency range. Moreover, in one embodiment, it can be provided that the amplitude of the electric voltage may vary dependent on the specific frequency of the frequency range. Therefore, the frequency sweep may not only comprise different frequencies, but it may also comprise respective different amplitudes.

For each frequency of the frequency sweep, a respective electric current is measured and considering the measured electric current, a respective electric impedance value is calculated. This is provided for all of the plural frequencies of the frequency sweep. So, for a specific turn of impedance spectroscopy, a set of electric impedance values can be achieved.

For generating the training data, the predetermined conditions may comprise a predefined temperature, a reference arrangement with regard to the electrodes, further predefined conditions and/or the like. Preferably, these predefined conditions shall preferably be constant during generating the training data.

Moreover, the water samples may be provided for a specific lime scale amount. However, it can also be provided to have the same water samples with different lime scale amounts on the electrodes. The impedance spectroscopy is preferably provided not only for one single water sample but also for a single water sample, wherein different lime scale amounts can occur. In this regard, a plurality of data sets can be generated which can be further processed in order to generate the training data.

With regard to the household appliance, which may be, for instance, a washing machine, a dishwasher, a coffee machine, or a steam oven or the like, the household appliance may comprise a lime scale removal module. The lime scale removal module may be configured to provide a detergent dispenser dispensing a respective detergent in order to allow enhancing the water hardness level. The detergent dispenser may be preferably an automatic detergent dispenser which is configured to dose an accurate amount of the detergent depending on the level of the water hardness. However, it may be also provided that the household appliance may assist manual dosing of the detergent. The appliance may have a display displaying information related to the water hardness level to a user. So, the user may be enabled dosing the detergent so that only a required dose of detergent will be added to the water.

The at least one component which is supplied with water during operation of the household appliance may be an electric heater, a tank, a pipeline and/or the like or any other component may be exposed to water.

It is further proposed that the supervised-learning is provided by a machine-learning algorithm using a neural network. The neural network allows a specific learning process so that the operation of the analysing apparatus can be improved during its use. The analysing apparatus can be based on a standard neural network so that it can be easily provided.

In this context, an artificial neural network can be understood as a software code or a compilation of several software code components, wherein the software code may comprise several software modules for different functions, for example one or more encoder modules and one or more decoder modules. An artificial neural network can be further understood as a non-linear model or algorithm that maps an input to an output, wherein the input is given by an input feature vector or an input sequence and the output may be an output category for a classification task or a predicted sequence. For example, the artificial neural network may be provided in a computer-readable way, for example, stored on a storage medium of the vehicle, in particular of the at least one computing unit. The neural network comprises several modules including the encoder module and the at least one decoder module. These modules may be understood as software modules or respective parts of the neural network. A software module may be understood as software code functionally connected and combined to a unit. A software module may comprise or implement several processing steps and/or data structures. The modules may, in particular, represent neural networks or sub-networks themselves. If not stated otherwise, a module of the neural network may be understood as a trainable and, in particular, trained module of the neural network. For example, the neural network and thus all of its trainable modules may be trained in an end-to-end fashion before the method is carried out. However, in other implementations, different modules may be trained or pre-trained individually. In other words, the method according to the invention corresponds to a deployment phase of the neural network.

In the context of the present disclosure, a water hardness level detection algorithm may be understood as a computer algorithm, which is able to identify one or more water hardness levels within a provided input dataset, for example input data, by specifying respective bounding boxes or regions of interest, ROI, and, in particular, assigning a respective water hardness level class to each of the bounding boxes, wherein the object classes may be selected from a predefined set of object classes. Therein, assigning a water hardness level class to a bounding box may be understood such that a corresponding confidence value or probability for the object identified within the bounding box being of the corresponding object class is provided. For example, the algorithm may provide such a confidence value or probability for each of the water hardness level classes for a given bounding box. Assigning the water hardness level class may for example include selecting or providing the water hardness level class with the largest confidence value or probability. Alternatively, the algorithm can specify only the bounding boxes without assigning a corresponding water hardness level class.

According to an exemplary embodiment, it is further proposed that, during a predetermined operation, back propagation is provided. The predetermined operation can be a specific portion of the operation of the household appliance, for example a finish of the operation, a maintenance or the like. It can be also manually actuated by the user. The back propagation can be achieved by using data which is acquired during the operation of the household appliance. This allows changing, especially updating, the analysing apparatus during its intended use.

According to another exemplary embodiment, it is further proposed that the impedance values are provided as complex impedance values. This embodiment considers that the impedance calculated may be not solely a resistance impedance. In this regard, training of the analysing apparatus as well as providing determining the water hardness level can be improved.

According to another exemplary embodiment, it is proposed that the frequency range extends at least between 0.1 Hz and about 1 MHz. The inventors found that this frequency range is well suited to provide proper information with regard to the water hardness level.

Moreover, it is further proposed that the frequency sweep is repeated dependent on a predefined condition of the household appliance and/or a predefined time distance. This allows automatically repeating determining the water hardness level. The predefined condition may be, for example, starting of the household appliance, starting of a program of the household appliance for a specific operation, a manual input of a user in the input device, and/or the like. The predefined time distance may be equidistant, for example, one or more month, one or more weeks, one or more days, one or more hours, combinations thereof, or the like. However, repeating the frequency sweep may be also not at a predefined time difference. The time difference may be varying from further conditions such as the last determined water hardness levels, or the like.

With regard to the second aspect, it is further proposed according to an exemplary embodiment that the analysing apparatus is configured to use a mathematic model based on supervised-learning which is trained with the training data, in order to enable the analyzing apparatus determining the water hardness when the electrodes are exposed to water. The supervised-learning allows a continuous improvement in determining the water hardness level. For this item, the training data can be updated by the time. Training can be repeated or an additional training can be provided.

According to another exemplary embodiment, it is proposed that generating the training data comprises a classification algorithm comprising at least a logistic regression or a support vector machine. With these classification algorithms the training process or the process to generate the training data can be improved.

According to another exemplary embodiment, it is proposed that generating the training data comprises principal component analysis. Principal component analysis can be used in exploratory data analysis and for making predictive models. It can be used for dimension reduction by projecting data, while preserving as much of the data's variation as possible.

According to yet another exemplary embodiment, it is further proposed that the predefined conditions comprise that lime deposition is provided for each of the water samples for at least five hours. The inventors found that this time interval is well suited to provide specific conditions for generating the training data. However, it can be provided that during this time, the impedance spectroscopy is repeated in order to receive different impedance spectroscopy for a specific one of the water samples depending on lime deposition. This embodiment considers that lime deposition may be time-dependent. Principal Component Analysis (PCA) is described for instance by G. H. Dunteman: Principal Component Analysis. Sage Publications, 1989, or L. Fahrmeir, A. Hamerle, G. Tutz (Hrsg.): Multivariate statistische Verfahren, New York 1996, or A. Handl, T. Kuhlenkasper: Multivariate Analysemethoden. Theorie und Praxis mit R, 3. Auflage. Springer, Berlin 2017, ISBN 978-3-662-54753-3.

According to another exemplary embodiment, it is further proposed that the frequency range extends at least between about 0.1 Hz and about 1 MHz. This preferably conforms to the frequency range with regard to the first aspect.

According to a fourth aspect of the present invention, there are provided one or more computer program products comprising computer-executable components which, when the program is run on a computer, are configured to carry out at least one of the respective methods as referred herein above. The above computer-program product may comprise computer-executable components which, when the program is run on a computer, perform the method aspects mentioned above in connection with the method aspects. The above computer program product may be embodied as a computer-readable storage medium.

The computer program product includes a program for a processing device which can be a computer, a computation unit, especially a digitized, preferably binary operating processing unit, or the like. The program includes instructions which, when executed on the processing device, perform the method according to the first and/or the second aspect. Consequently, the computer program product comprises, according to an exemplary embodiment, a computer-readable medium, on which software code portions of the computer program product are stored. The computer-readable medium may be a storage device, such as a RAM, a CD, a DVD, combinations thereof, or the like. According to a further aspect, the program product is directly loadable into an internal memory of the processing device. The internal memory may be, for example, a RAM or the like.

A computer may in particular be understood as a data processing device, which comprises processing circuitry. The computer can therefore in particular process data to perform computing operations. This may also include operations to perform indexed accesses to a data structure, for example a look-up table, LUT. In particular, the computer may include one or more computers, one or more microcontrollers, and/or one or more integrated circuits, for example, one or more application-specific integrated circuits, ASIC, one or more field-programmable gate arrays, FPGA, and/or one or more systems on a chip, SoC. The computer may also include one or more processors, for example one or more microprocessors, one or more central processing units, CPU, one or more graphics processing units, GPU, and/or one or more signal processors, in particular one or more digital signal processors, DSP. The computer may also include a physical or a virtual cluster of computers or other of said units. In various embodiments, the computer includes one or more hardware and/or software interfaces and/or one or more memory units. A memory unit may be implemented as a volatile data memory, for example a dynamic random access memory, DRAM, or a static random access memory, SRAM, or as a nonvolatile data memory, for example a read-only memory, ROM, a programmable read-only memory, PROM, an erasable programmable read-only memory, EPROM, an electrically erasable programmable read-only memory, EEPROM, a flash memory or flash EEPROM, a ferroelectric random access memory, FRAM, a magnetoresistive random access memory, MRAM, or a phase-change random access memory, PCRAM.

The invention has been explained with regard to various claim categories. If not stated differently, features and/or advantages described with regard to one claim category can be applied analogously to all other claim categories.

The teaching of the present invention can be readily understood and at least some additional specific details will appear by considering the following detailed description of at least one exemplary embodiment in conjunction with the accompanying drawings, wherein the drawings show the following:

Fig. 1 a schematic front view of a washing machine;

Fig. 2 a schematic view of two electrodes arranged to contact water;

Fig. 3 a schematic view as fig. 2, wherein lime scale is deposited on one of the electrodes;

Fig. 4 two schematic diagrams indicating the inventive concept;

Fig. 5 a schematic block diagram of a control apparatus of the washing machine according to fig. 1;

Fig. 6 a schematic diagram of an absolute value of a complex impedance calculated according to the invention;

Fig. 7 a schematic diagram of a phase angle of the complex impedance calculated according to the invention;

Fig. 8 a schematic side view of the two electrodes according to fig. 2 without lime scale deposition showing an electric field;

Fig. 9 a schematic circuit diagram showing an electrical performance of the electrodes according to fig. 8;

Fig. 10 a schematic side view of the two electrodes according to fig. 2 with lime scale deposition showing an electric field;

Fig. 11 a schematic circuit diagram showing an electrical performance of the electrodes according to fig. 10;

Fig. 12 a schematic diagram showing the weight of lime scale deposition versus a water hardness level;

Fig. 13 a schematic diagram showing an electric impedance value versus the weight of lime scale deposition;

Fig. 14 a schematic view combining the diagrams according to figs. 12 and 13 in order to receive a schematic diagram showing a dependency of the electric impedance value versus the water hardness level;

Fig. 15 through fig. 19 different lime scale depositions on one of the electrodes dependent on the water hardness level;

Fig. 20 a schematic diagram showing a classification based on Principal Component Analysis;

Fig. 21 a schematic block diagram showing generating of training data for an analysis apparatus of a control apparatus of the washing machine according to fig. 1; and

Fig. 22 a schematic diagram as fig. 20, wherein three water samples having different water hardness levels are used to generate training data.

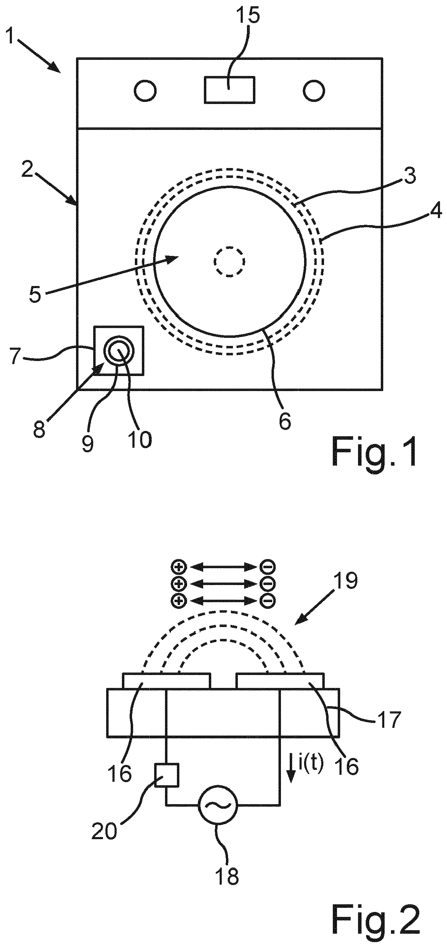

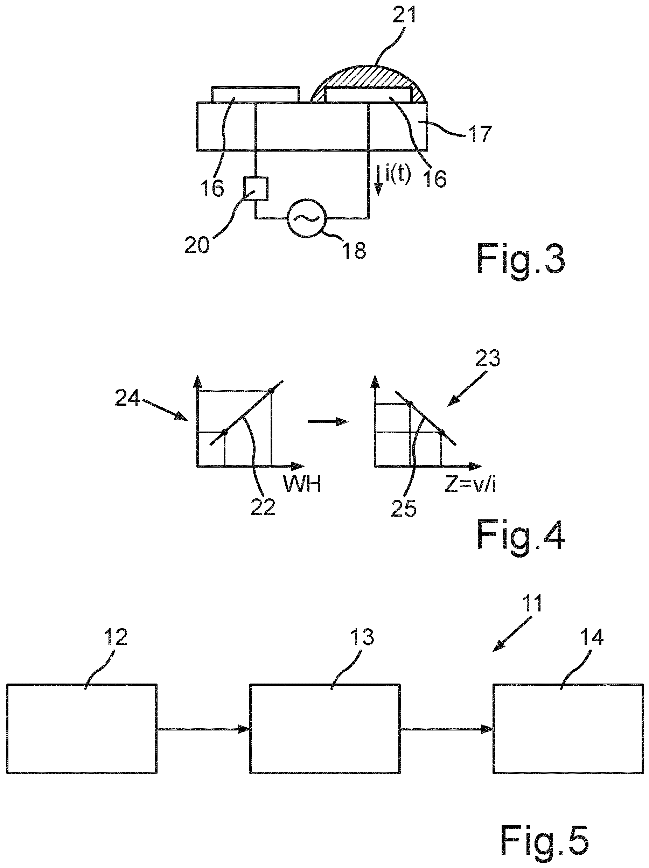

Fig. 1 shows a simplified schematic front view of a household appliance 1 for the care of laundry items, which may be, for example, a washing machine or a washer-dryer. The household appliance 1 comprises a housing 2, in which a laundry drum 3 is rotatably mounted. A longitudinal axis or axis of rotation of the laundry drum 3 is oriented perpendicular to the plane of the figure in this embodiment as shown in Fig. 1. The household appliance 1 further comprises a lye container 4, which is also arranged in the housing 2 and surrounds the laundry drum 3. The laundry drum 3 comprises a hollow-cylindrical casing and a rear end wall and is furthermore open at the front, so that a loading opening is provided here, via which laundry items can be introduced into an interior 5 of the laundry drum 3. The loading opening can be closed by a door 6, which is arranged on the housing 2 such that it can be moved, especially pivoted, for opening. The washing machine 1 comprises a control apparatus 11 (fig. 5) which, in turn, comprises a display 15 in order to indicate specific information related to operation of the washing machine 1 to a user. The control apparatus 11 is configured to control the operation of the washing machine, and especially, its components, as detailed below.

Furthermore, the household appliance 1 comprises a pump 7, which is shown only schematically at present. The pump 7 can be used, for example, to convey the washing suds. The pump 7 comprises an electric machine 8, which in turn comprises a stator 9 and a rotor 10, which are shown only schematically at present. The electric machine 8 is designed as a permanently excited synchronous machine. The rotor 10 has a plurality of permanent magnets.

Fig. 5 shows an exemplary section of the control apparatus 11 of the washing machine 1 according to fig. 1. The control apparatus 11 comprises a precipitation module 12, an analysing apparatus 13 as well as a lime scale removal module 14. The control apparatus 11, among others, refers to a system capable of determining the water hardness level of the washing machine 1. This system allows self-configuration of operation of the washing machine 1 depending on two different adjustable operation modes:

1. Providing an accurate automatical dosing of an amount of a detergent depending on a determined water hardness level.

2. It is also possible to provide a manual dosing by a user, wherein the washing machine 1 displays information related to the water hardness level to the user via a display 15 of the washing machine 1 so that the user knows or can determine a required amount of the detergent to be dosed. This detergent may be put into the lye container 4 by the user.

Especially, the invention relates to the analysing apparatus 13.

The operation of the analysing apparatus 13 is especially based on electrochemical impedance spectroscopy. This technique allows determining an electric impedance level between two electrodes with regard to a specific frequency range which is usually provided as a frequency sweep. In this embodiment, the frequency range extends from about 0.1 Hz trough about 1 MHz. In alternative embodiments, the frequency range may be varying. For the purpose of the invention, in this embodiment, it is proposed that plural specific different frequencies of the frequency range are used to establish the frequency sweep.

Fig. 2 shows a general schematic view of an arrangement of two electrodes 16 which are arranged to contact water in the washing machine 1. As can be seen from the schematic drawing of fig. 2, the electrodes 16 which are usually provided by a specific electric conductive material such as metal, especially stainless steel, copper, alloys thereof, or the like, which may be fixed to a substrate 17 which can be a wall of the lye container, or a specific plate of non-conducting material which may be arranged inside of the lye container, or the like. It may also be a water pipe of the washing machine 1.

The electrodes 16 are connected via electric conductors with a voltage source 18. One of the conductors is provided with an electric current sensor 20. Once the voltage source 18 provides an electric voltage between the both electrodes 16, an electric field will be present between the electrodes 16 indicated by electric field lines 19 in Fig. 2, and an electric current will flow in response to the electric voltage. This electric current is measured with the current sensor 20.

Fig. 3 shows a schematic view as fig. 2, wherein a lime scale 21 is deposited on one of the electrodes 16. The lime scale 21 will affect the electric field so that determining an electric conductivity between the electrodes 16 could not be sufficient in order to determine the water hardness level with a predetermined accuracy. This is further indicated by fig. 4 showing two schematic diagrams 23, 24. In the diagram 24, an ordinate is allocated to a lime scale deposition, wherein an abscissa is allocated to a water hardness level. A graph 22 shows that the relationship between the lime scale deposition and the water hardness level.

Diagram 23 of fig. 4 shows an ordinate which is also allocated to the lime scale deposition, wherein an abscissa is allocated to an electric impedance level. The electric impedance level is calculated based on the electric voltage acting on the electrodes and the current measured by the current sensor 20.

A graph 25 shows the dependency. As can be seen from fig. 4, the electric impedance value depends on a composition of the media and, therefore, the electric current measured in response to an electric voltage acting on the electrodes 16 is usually affected by ions of the medium, contacting the electrodes 16 which is in this case the water in the washing machine 1 combined with lime scale deposition. This means that all ions of the water will affect the response resulting in an inaccurate determination of the water hardness level due to the presence of other ions that do not affect the water hardness but do affect the electric impedance level.

According to the invention, this problem is overcome by considering the amount of lime deposition on surfaces of the electrodes 16 so that lime deposition can be considered by determining the water hardness level.

The electric impedance between the electrodes 16 is, according to the present embodiment, measured by applying an electric alternating voltage between the electrodes 16. During this operation, the frequency of the alternating electric voltage and, if necessary, also an amplitude of the alternating electric voltage, can be varied. In the present embodiment, the amplitude is substantially constant. However, in alternative embodiments, the amplitude can be also frequency dependent.

In the present embodiment, a frequency sweep is provided with the alternating electric voltage. In response to the alternating electric voltage acting on the electrodes 16, a respective electric current is measured with the current sensor 20.

With regard to impedance spectroscopy technique, an excitation signal can be of the form

$$E_t = E_0 \sin(\omega_t + \varphi)$$

where $E_t$ refers to potential at a time t, Eo refers to an amplitude of the signal, and $\omega$ refers to

$$\omega = 2\pi f$$

where f refers to the frequency. This formula can be applied to the alternating electric voltage. With regard to the measured current, the response signal which is presently the electric current measured with the current sensor 20, conforms usually to the following equation:

$$I_t = I_0 \sin(\omega_t + \varphi)$$

Wherein $\varphi$ is a phase difference with regard to the phase of the alternating electric voltage.

The electric impedance can be derived by using Ohm's law as follows:

$$Z = \frac{E_I}{I_t} = \frac{E_0 \sin(\omega t)}{I_0 \sin(\omega t + \varphi)} = Z0 \frac{\sin(\omega t)}{\sin(\omega t + \varphi)}$$

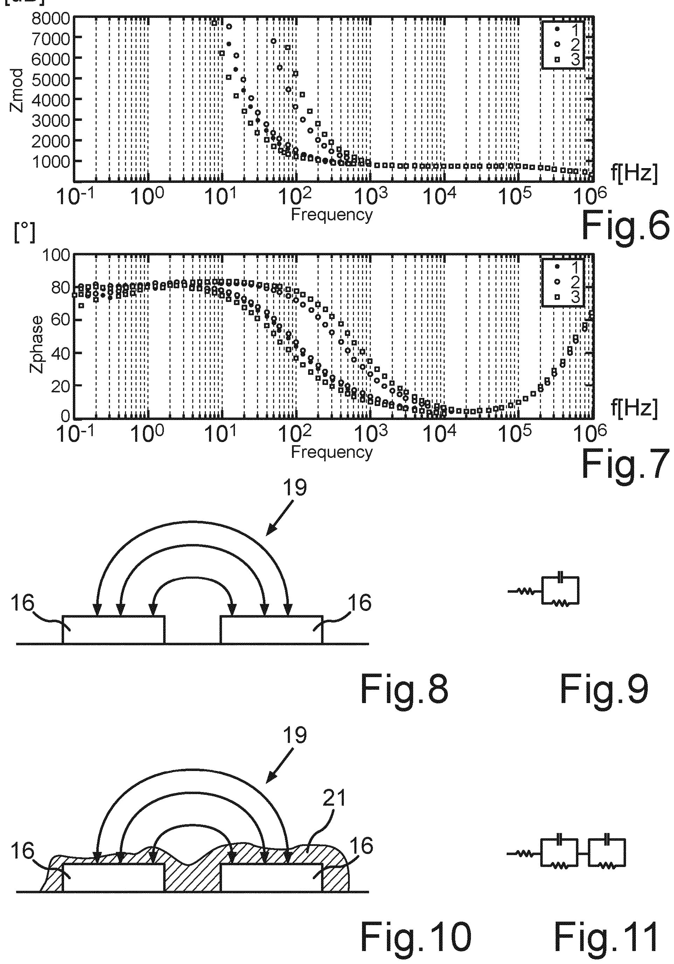

For a specific water sample, the impedance spectroscopy leads to a data set for plural different frequencies of the frequency sweep which result is shown for three different water hardness levels of the water according to fig. 6 and 7.

Fig. 6 shows a schematic diagram of an absolute value of the electric impedance calculated by the formula above under consideration of providing an impedance spectroscopy as discussed above. Fig. 7 shows a diagram of a phase angle of the impedance as mentioned before. Both diagrams show the dependencies on the frequency. They form a Bode-diagram.

In both diagrams of fig. 6 and 7 the abscissa is related to the frequency. In fig. 6, the ordinate is allocated to the absolute value of the electric impedance calculated, wherein in fig. 7 the ordinate is allocated to the phase angle of the calculated electric impedance. As can be seen from fig. 6 and 7, the impedance spectroscopy allows an indication with regard to the water hardness level.

In this regard, fig. 8 and 10 show the general situation for the washing machine 1 in respective side views with the electrodes. Consequently, fig. 9 und 11 refer to respective equivalent circuit diagrams with regard to the respective electric impedances provided by the different situations according to fig. 8 and 10. As can be seen from fig. 9 and 11, the lime deposition 21 provides an additional influence on the electric impedance between the both electrodes 16. This is the reason, why impedance spectroscopy itself may not be sufficient to determine the water hardness level with high accuracy.

However, the invention takes into account the three step procedure as detailed related to fig. 5, namely the position or precipitation, respectively, detection or analysing respectively, and removal. Analysing with the analysing apparatus according to the second step as shown in fig. 5, leads to an indirect determination of the water hardness level considering lime scale deposited on the electrode surfaces.

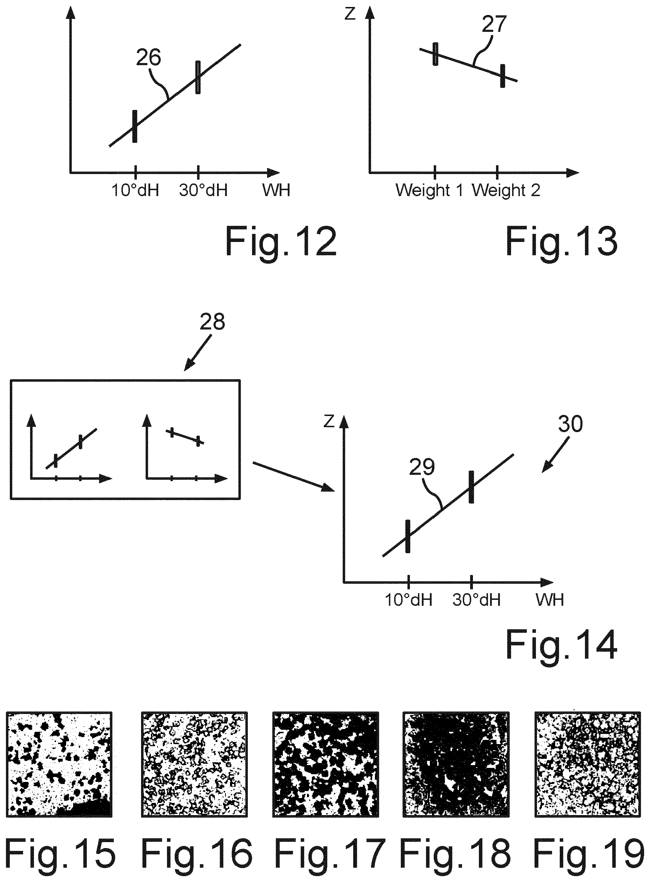

According to the invention, a first step is to observe a correlation between the water hardness level and the amount of lime scale deposited on the electrodes 16. A second step is directed to observing a correlation between the amount of the lime scale deposited on the electrode 16 and the electric impedance values. A third step relates to use the first and the second step to find a correlation between the water hardness level and the electric impedance values. These steps are illustrated by the fig. 12 through 14. In fig. 12, the ordinate is allocated to a lime scale weight and the abscissa is allocated to the water hardness level. A graph 26 shows the correlation.

In fig. 13, the diagram shows an ordinate which is allocated to the electric impedance value, wherein an abscissa is allocated to a lime scale weight. A graph 27 shows the dependency. In fig. 14, a diagram 28 shows the fig. 12 and 13 which are processed in order to receive a diagram 30, in which an ordinate is allocated to the electric impedance value and the abscissa is allocated to the water hardness level. A graph 29 shows the resulted dependency.

In order to confirm the dependency between the water hardness level and the amount of lime scale deposited on the electrodes 16, a scanning electron microscope (SEM) can be used. In the fig. 15 through 19, a coverage level of the electrodes 16 with lime scale deposition depending on the water hardness level of the water is shown. As can be seen from fig. 15 through 19, a high water hardness level is connected with a high coverage level. The duration of deposition of lime scale at the different water hardness levels according to fig. 15 through 19 was the same. In Fig. 15, the water hardness level is about 10°dH, in Fig. 16, the water hardness level is about 15°dH, in Fig. 17, the water hardness level is about 20°dH, in Fig. 18, the water hardness level is about 25°dH, and in Fig. 19, the water hardness level is about 30°dH.

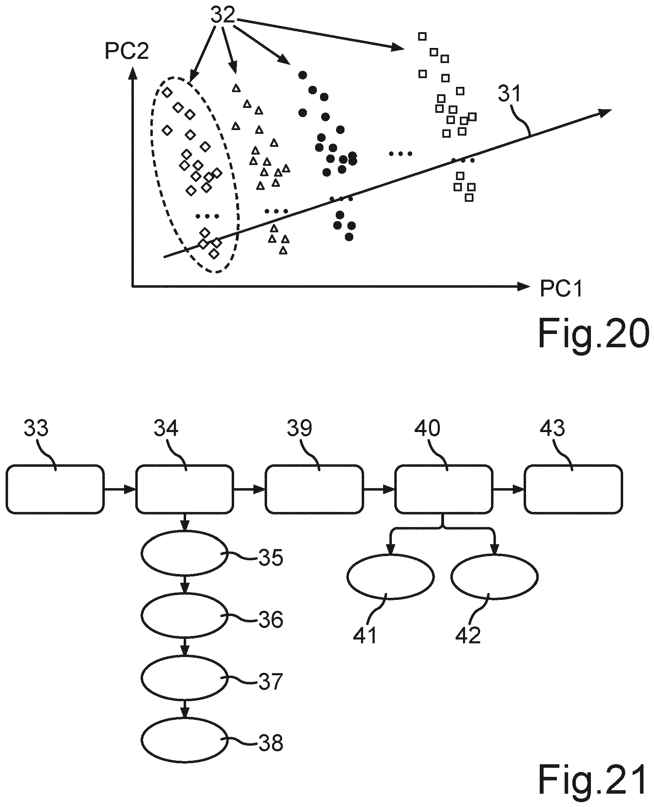

Regarding the impedance spectroscopy technique, these samples according to the fig. 15 through 19 show that the different lime deposition may have also different effects on the electric field which, in response, also affects the electric impedance. Fig. 20 shows a classification using the data according to the fig. 15 through 19. This classification is based on the calculated electric impedance data received by impedance spectroscopy.

In this regard, a principal component analysis (PCA) is used. As can be seen from Fig. 20, an ordinate is allocated to PC2, wherein an abscissa is allocated to PC1. This technique is based on singular value decomposition. As can be seen from Fig. 20, a graph 31 is allocated to the water hardness level. In this diagram, point clouds 32 can be achieved, wherein a specific point cloud 32 can be allocated to a specific water hardness level range. In this regard, points belonging to the same water hardness level can be grouped, wherein PC1 represents the trend of the electric impedance correlated to the water hardness level. Generally, the analysis has common steps whether machine-learning or deep-learning techniques are being applied.

A point cloud may be understood as a set of points, wherein each point is characterized by respective coordinates in a two-dimensional, or three-dimensional, or higher dimensional coordinate system. In case of a three-dimensional point cloud, the three-dimensional coordinates may, for example, be determined by the direction of incidence of the reflected components. In other words, the three-dimensional coordinate system may be a three-dimensional polar coordinate system. However, the information may also be pre-processed to provide three-dimensional Cartesian coordinates for each of the points.

In general, the points of a point cloud may be given in an orderless or unsorted manner, in contrast to, for example, a camera image. In addition to the information, namely the two-dimensional or three-dimensional coordinates, the point cloud may also store additional information or measurement values for the individual points such as an frequency-dependent characteristics of the water, of lime, of any respective sensor signal.

This is further explained with regard to Fig. 21, showing a schematic block diagram for generating of training data of an analysis apparatus 13 of the control apparatus 11 of the washing machine 1 according to fig. 1.

The before-mentioned steps can be considered as data adaption in order to enable data processing. These steps preferably include data cleaning, data reshape, data normalisation and further techniques. In this regard, according to fig. 21, a step 33 relates to data collection and studying of them. A next step 34 refers to data processing. Data processing may include determining the raw data 35, provide data correction 36, provide data normalisation 37 and provide reshape 38. Then, further extraction and selection is provided at 39. At 40, the model is selected, which may be regression 41 or a classification 42. Finally, test, train and validation at 43 is provided.

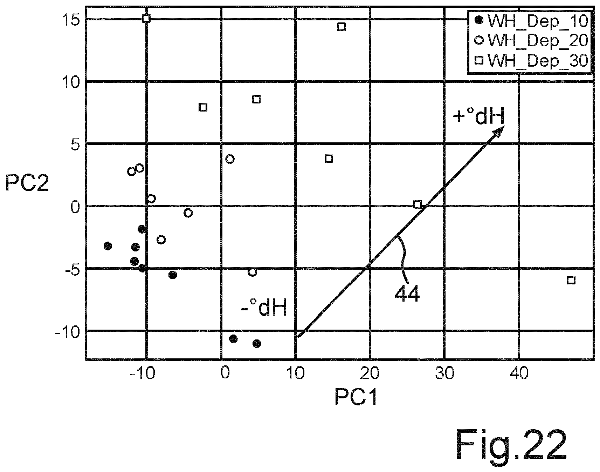

One step, after calculating electric impedance, is the use of the principal component analysis. PCA is used in exploratory data analysis and for making predictive models. It is moreover commonly used for dimension reduction by projecting each data point onto only first few principle components in order to obtain a lower-dimensional data while preserving as much of the data's variation as possible. A first principle component can equivalently be defined as a direction that maximises the variants of the projected data. An i-th principle component (PCi) can be taken as a direction orthogonal to the first principle component that maximises the variants of the projected data. For receiving training data, a PCA score plot according to fig. 22 is shown. According to fig. 22, there are shown samples that have lime scale deposited during five hours using three synthetic water solution samples of 10°dH, 20°dH and 30°dH. Afterwards, electric impedances of these samples are measured at water having a water hardness level of 20°dH. These representation serves to observe data correlation and trends. In fig. 22, the ordinate is allocated to PC2, wherein the abscissa is allocated to PC1. A graph 44 shows the trend related to the water hardness level. In the present embodiment, it is clear how the data points shown in fig. 22 representing electric impedance values in the whole frequency range are moving from one region to another region depending on the water hardness level of the water. This same behaviour has been observed in the fig. 15 through 19. This is a clear indication that the machine-leaning methods combined with the impedance spectroscopy is a valid method for determining the water hardness level of water samples.

After having confirmed the possibility of determining, the two classification algorithms 41 and 42 can be used. These two classification algorithms are classical algorithms that are well known to those skilled in the art. In the present embodiment, the data is split into training data, test data and validation data according to 43 in fig. 21. The following table shows the classification accuracy level obtained when training logistic regression and support vector machine (SVM) classification algorithms with two different data splits.

| | Measurement | Logistic Regression | SVM Lineal |
|---|---|---|---|
| Train: 80% Val: 20% | 10°dH | 60% | 100% |
| | 20°dH | 80% | 80% |
| | 30°dH | 100% | 80% |
| Train: 50% Val: 50% | 10°dH | 81.80% | 90.90% |
| | 20°dH | 91.70% | 83.30% |
| | 30°dH | 75% | 75% |

As can be seen from the table above, the result indicates the high potential of the inventive method for determining the water hardness level of water samples with high accuracy.

Generally, the invention may be connected with the following advantages:

- accurate water hardness determination in home appliances;
- lime scale deposition prevention (due to an automatic home appliance configuration);
- longer home appliance lifetime;
- lower water, detergents and energy costs (due to washing processes using softened water);
- environmental friendliness (less overdosage of detergents);
- the use of novel materials and the lime scale removal module allow for a robust and durable system;
- cost: microelectronics fabrication methods allow for a considerable reduction of the material costs;
- no need of reagents for sensor operation.

The embodiments detailed above shall only be understood as further explaining the invention and not to limit the scope of the claims.

**List of Reference Characters**

1    washing machine
2    housing
3    laundry drum
4    lye container
5    interior
6    door
7    pump
8    electric machine
9    stator
10   rotor
11   control apparatus
12   precipitation module
13   analysing apparatus

| 14 | lime scale removal module |
|---|---|
| 15 | display |
| 16 | electrode |
| 17 | substrate |
| 18 | voltage source |
| 19 | electric field lines |
| 20 | electric current sensor |
| 21 | lime scale |
| 22 | graph |
| 23 | diagram |
| 24 | diagram |
| 25 | graph |
| 26 | graph |
| 27 | graph |
| 28 | diagram |
| 29 | graph |
| 30 | diagram |
| 31 | graph |
| 32 | point cloud |
| 33 | data collection and study |
| 34 | data processing |
| 35 | raw data |
| 36 | data correction |
| 37 | data normalization |
| 38 | reshape |
| 39 | feature extraction |
| 40 | select model |
| 41 | regression |
| 42 | classification |
| 43 | train, test, validation |
| 44 | graph |

**Claims**

1. A method of determining a water hardness level of water, especially for determining the water hardness of water used by a household appliance (1), wherein

   - at least two electrodes (16) are exposed to the water;
   - an electric voltage acts on the at least two electrodes (16), wherein the electric voltage is an alternating electric voltage having a predetermined frequency range, wherein the frequency range is provided by a frequency sweep comprising plural different frequencies of the frequency range;
   - in response to the electric voltage acting on the electrodes (16), respective electric currents are measured for the different frequencies of the frequency sweep, and
   - respective electric impedance values are calculated for the plural frequencies of the frequency sweep based on the electric voltage and the electric currents measured,

   **characterized in that**
   the electric impedance values are processed by an analyzing apparatus (13) connected with the electrodes (16) which is configured to use a machine-learning model based on supervised learning, in order to determine the water hardness level.

2. The method according to claim 1, **characterized in that** the supervised learning is provided by a machine-learning algorithm using a neural network.

3. The method according to one of the preceding claims, **characterized in that**, during a predetermined operation, back propagation is provided.

4.  The method according to one of the preceding claims, **characterized in that** the impedance values are provided as complex impedance values.

5.  The method according to one of the preceding claims, **characterized in that** the frequency range extends at least between 0.1 Hz and 1 MHz.

6.  The method according to one of the preceding claims, **characterized in that** the frequency sweep is repeated dependent on a predefined condition of the household appliance (1) and/or a predefined time distance.

7.  A method of training an analyzing apparatus (13), especially an analyzing apparatus (13) as used according to one of the preceding claims, wherein training data is generated by using the analyzing apparatus (13) under predefined conditions, wherein the method comprises the steps of

    - providing different water samples having water hardness levels differing from each other,
    - for each of the different water samples,

        - measuring an amount of lime deposition on electrodes (16) connected with the analyzing apparatus (13) dependent on the different water samples under the predefined conditions;
        - subjecting the (16) electrodes to an electric voltage, wherein the electric voltage is an alternating electric voltage having a predetermined frequency range, wherein the frequency range is provided by a frequency sweep comprising plural different frequencies of the frequency range,
        - measuring electric currents responsive to the electric voltage for each of the plural different frequencies of the frequency sweep; and
        - calculating electric impedances dependent on the plural different frequencies of the frequency sweep resulting in respective plural frequency dependent electric impedance values,

    - processing the different water samples and the respective lime deposition amounts (21) in order to identify a correlation between the different water samples and the respective lime deposition amounts (21);
    - processing the plural frequency dependent electric impedance values of the frequency sweeps and the respective lime deposition amounts (21), which depend on the water samples, in order to identify a correlation between the plural frequency dependent electric impedance values of the frequency sweeps and the respective lime deposition amounts (21) dependent on the water hardness levels of the different water samples;
    - generating the training data based on the identified correlation between the plural frequency dependent electric impedance values of the frequency sweeps and the respective different water samples in order to train the analyzing apparatus (21); and
    - applying the training data on a machine-learning model using supervised learning.

8.  The method according to claim 7, **characterized in that** the analyzing apparatus (13) is configured to use a mathematic model based on supervised learning which is trained with the training data, in order to enable the analyzing apparatus (13) to determine the water hardness when the electrodes are exposed to water.

9.  The method according to claim 7 or 8, **characterized in that** generating the training data comprises a classification algorithm comprising at least a logistic regression or a support vector machine.

10. The method according to one of the claims 7 through 9, **characterized in that** generating the training data comprises principal component analysis.

11. The method according to one of the claims 8 through 10, **characterized in that** the predefined conditions comprise that lime deposition (21) is provided for each of the water samples for at least five hours.

12. The method according to one of the claims 8 through 11, **characterized in that** the frequency range extends at least between 0.1 Hz and 1 MHz.

13. A computer program product comprising instructions, which, when the computer program is executed by a computer, cause the computer to carry out the method of one of the preceding claims.

14. A computer readable medium comprising instructions which, when executed by a computer, cause the computer to carry out the method of one of the preceding claims 1 through 12.

15. A household appliance (1), especially a washing machine, a dishwasher, a coffee machine, or a steam oven, having at least one component, which is supplied with water during operation of the household appliance (1), at least two electrodes (16) which are arranged to contact the water, a current sensor (20) for sensing an electric current passing the electrodes (16) and a control apparatus (11) configured to control the operation of the household appliance (1), wherein the control apparatus (11) is electrically connected with the at least two electrodes (16) and the control apparatus (11) is configured to determine a water hardness level of the water, **characterized in that** the control apparatus (11) comprises an analyzing apparatus (13) connected with the electrodes (16) which is configured to use a machine-learning model based on supervised learning, in order to determine the water hardness level, wherein the control apparatus (11) is further configured to

- supply an electric voltage to the at least two electrodes (16), wherein the electric voltage is an alternating electric voltage having a predetermined frequency range, wherein the frequency range is provided by a frequency sweep comprising plural different frequencies of the frequency range;
- in response to the electric voltage acting on the electrodes (16), measure electric currents with the current sensor (20) responsive to the electric voltage for each of the plural different frequencies;
- calculate respective electric impedance values for the plural frequencies of the frequency sweep based on the electric voltage and the electric currents measured; and
- process the electric impedance values by the analyzing apparatus (13) connected with the electrodes (16) in order to determine the water hardness level.

**Amended claims in accordance with Rule 137(2) EPC.**

1. A method of determining a water hardness level of water, especially for determining the water hardness of water used by a household appliance (1), wherein

- at least two electrodes (16) are exposed to the water;
- an electric voltage acts on the at least two electrodes (16), wherein the electric voltage is an alternating electric voltage having a predetermined frequency range, wherein the frequency range is provided by a frequency sweep comprising plural different frequencies of the frequency range;
- in response to the electric voltage acting on the electrodes (16), respective electric currents are measured for the different frequencies of the frequency sweep, and
- respective electric impedance values are calculated for the plural frequencies of the frequency sweep based on the electric voltage and the electric currents measured,

**characterized in that**
the electric impedance values are processed by an analyzing apparatus (13) connected with the electrodes (16) which is configured to use a machine-learning model based on supervised learning, in order to determine the water hardness level, wherein processing comprises:

- in a first step, observing a correlation between the water hardness level and the amount of lime scale deposited on the electrodes (16);
- in a second step, observing a correlation between the amount of the lime scale deposited on the electrodes (16) and the electric impedance values; and
- in a third step, using the first and the second step to find a correlation between the water hardness level and the electric impedance values.

2. The method according to claim 1, **characterized in that** the supervised learning is provided by a machine-learning algorithm using a neural network.

3. The method according to one of the preceding claims, **characterized in that**, during a predetermined operation, back propagation is provided.

4. The method according to one of the preceding claims, **characterized in that** the impedance values are provided as complex impedance values.

5. The method according to one of the preceding claims, **characterized in that** the frequency range extends at least between 0.1 Hz and 1 MHz.

6. The method according to one of the preceding claims, **characterized in that** the frequency sweep is repeated dependent on a predefined condition of the household appliance (1) and/or a predefined time distance.

7. A method of training an analyzing apparatus (13), especially an analyzing apparatus (13) as used according to one of the preceding claims, wherein training data is generated by using the analyzing apparatus (13) under predefined conditions, wherein the method comprises the steps of

   - providing different water samples having water hardness levels differing from each other,
   - for each of the different water samples,

      - measuring an amount of lime deposition on electrodes (16) connected with the analyzing apparatus (13) dependent on the different water samples under the predefined conditions;
      - subjecting the (16) electrodes to an electric voltage, wherein the electric voltage is an alternating electric voltage having a predetermined frequency range, wherein the frequency range is provided by a frequency sweep comprising plural different frequencies of the frequency range,
      - measuring electric currents responsive to the electric voltage for each of the plural different frequencies of the frequency sweep; and
      - calculating electric impedances dependent on the plural different frequencies of the frequency sweep resulting in respective plural frequency dependent electric impedance values,

   - processing the different water samples and the respective lime deposition amounts (21) in order to identify a correlation between the different water samples and the respective lime deposition amounts (21);
   - processing the plural frequency dependent electric impedance values of the frequency sweeps and the respective lime deposition amounts (21), which depend on the water samples, in order to identify a correlation between the plural frequency dependent electric impedance values of the frequency sweeps and the respective lime deposition amounts (21) dependent on the water hardness levels of the different water samples;
   - generating the training data based on the identified correlation between the plural frequency dependent electric impedance values of the frequency sweeps and the respective different water samples in order to train the analyzing apparatus (21); and
   - applying the training data on a machine-learning model using supervised learning.

8. The method according to claim 7, **characterized in that** the analyzing apparatus (13) is configured to use a mathematic model based on supervised learning which is trained with the training data, in order to enable the analyzing apparatus (13) to determine the water hardness when the electrodes are exposed to water.

9. The method according to claim 7 or 8, **characterized in that** generating the training data comprises a classification algorithm comprising at least a logistic regression or a support vector machine.

10. The method according to one of the claims 7 through 9, **characterized in that** generating the training data comprises principal component analysis.

11. The method according to one of the claims 8 through 10, **characterized in that** the predefined conditions comprise that lime deposition (21) is provided for each of the water samples for at least five hours.

12. The method according to one of the claims 8 through 11, **characterized in that** the frequency range extends at least between 0.1 Hz and 1 MHz.

13. A computer program product comprising instructions, which, when the computer program is executed by a computer, cause the computer to carry out the method of one of the preceding claims when the computer program product is run on a control apparatus (11) of a household appliance (1) according to claim 15.

14. A computer readable medium comprising instructions which, when executed by a computer, cause the computer to carry out the method of one of the preceding claims 1 through 12 when the computer program product is run on a control apparatus (11) of a household appliance (1) according to claim 15.

15. A household appliance (1), especially a washing machine, a dishwasher, a coffee machine, or a steam oven, having at least one component, which is supplied with water during operation of the household appliance (1), at least two electrodes (16) which are arranged to contact the water, a current sensor (20) for sensing an electric current passing the electrodes (16) and a control apparatus (11) configured to control the operation of the household appliance (1), wherein the control apparatus (11) is electrically connected with the at least two electrodes (16) and the control apparatus (11) is configured to determine a water hardness level of the water,

**characterized in that** the control apparatus (11) comprises an analyzing apparatus (13) connected with the electrodes (16) which is configured to use a machine-learning model based on supervised learning, in order to determine the water hardness level, wherein the control apparatus (11) is further configured to

- supply an electric voltage to the at least two electrodes (16), wherein the electric voltage is an alternating electric voltage having a predetermined frequency range, wherein the frequency range is provided by a frequency sweep comprising plural different frequencies of the frequency range;
- in response to the electric voltage acting on the electrodes (16), measure electric currents with the current sensor (20) responsive to the electric voltage for each of the plural different frequencies;
- calculate respective electric impedance values for the plural frequencies of the frequency sweep based on the electric voltage and the electric currents measured; and
- process the electric impedance values by the analyzing apparatus (13) connected with the electrodes (16) in order to determine the water hardness level, wherein processing comprises:

- in a first step, observing a correlation between the water hardness level and the amount of lime scale deposited on the electrodes (16);
- in a second step, observing a correlation between the amount of the lime scale deposited on the electrodes (16) and the electric impedance values; and
- in a third step, using the first and the second step to find a correlation between the water hardness level and the electric impedance values.

Fig.1

Fig.2

Fig.3

Fig.4

Fig.5

Fig.6

Fig.7

Fig.8

Fig.9

Fig.10

Fig.11

Fig.12

Fig.13

Fig.14

Fig.15  Fig.16  Fig.17  Fig.18  Fig.19

Fig.20

Fig.21

Fig.22

Europäisches
Patentamt

European
Patent Office

Office européen
des brevets

# EUROPEAN SEARCH REPORT

Application Number

EP 22 38 2988

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 944 764 A1 (BSH HAUSGERAETE GMBH [DE]; BSH ELECTRODOMESTICOS ESPANA SA [ES]) 2 February 2022 (2022-02-02)<br>* abstract *<br>* paragraph [0051] – paragraph [0086] *<br>* figures 1-3 *<br>----- | 1-15 | INV.<br>G01N33/18 |
| A | US 11 397 159 B1 (SHAPIRO DANIEL [CA] ET AL) 26 July 2022 (2022-07-26)<br>* abstract *<br>* column 2 – column 4 *<br>----- | 1-15 | |

TECHNICAL FIELDS
SEARCHED      (IPC)

G01N

The present search report has been drawn up for all claims

| Place of search | Date of completion of the search | Examiner |
|---|---|---|
| The Hague | 21 February 2023 | Appeltant, Lennert |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
.................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 22 38 2988

This annex lists the patent family members relating to the patent documents cited in the above-mentioned European search report.
The members are as contained in the European Patent Office EDP file on
The European Patent Office is in no way liable for these particulars which are merely given for the purpose of information.

21-02-2023

| Patent document cited in search report | | Publication date | Patent family member(s) | | Publication date |
|---|---|---|---|---|---|
| EP 3944764 | A1 | 02-02-2022 | CN 114002745 A | | 01-02-2022 |
| | | | EP 3944764 A1 | | 02-02-2022 |
| US 11397159 | B1 | 26-07-2022 | NONE | | |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5707246 A

**Non-patent literature cited in the description**

- **G. H. DUNTEMAN.** Principal Component Analysis. Sage Publications, 1989
- Multivariate statistische Verfahren. 1996
- **A. HANDL ; T. KUHLENKASPER.** Multivariate Analysemethoden. Theorie und Praxis mit R. Springer, 2017